# EUROPEAN PATENT APPLICATION

(11) **EP 4 544 985 A1**
(43) Date of publication of application: **30.04.2025**
(21) Application number: 24204894.0
(22) Date of filing: 07.10.2024
(51) Int. Cl.: A61B 1/303, A61B 1/31, A61B 1/32, A61B 1/00

(54) **DEVICE FOR INSPECTING A CAVITY OF A PATIENT**

(30) Priority: 26.10.2023 IT 202300022464
(71) Applicant: THD S.p.A., 42015 Correggio, Reggio Emilia (IT)
(72) Inventor: BASTIA, FILIPPO, 41019 SOLIERA (IT)
(74) Representative: Paparo, Aldo

(57) **Abstract**

Device for inspecting a cavity of a patient, comprising a dilator body, extending along a longitudinal axis between a distal end and a proximal end, a handle, applied or fixed to the proximal end of the dilator body. The device comprises a distal portion having a substantially tubular shape and a proximal portion extending from the distal portion in a diverging fashion, wherein the distal portion defines a zone of dilation of an internal cavity of a patient and delimits an internal volume accessible from a rear access opening made on the proximal portion.

The distal portion of the dilator body comprises at least one front opening to enable access to the patient's mucosa or tissue from said internal volume.

The device comprises at least one arm mounted on the proximal portion and comprising at least one rest seat configured to receive, resting upon it, a front surface of a laser head and to enable the movement thereof in such a way as to emit a linear laser beam through said front opening; wherein said at least one arm is rotatable according to a main direction of movement relative to said proximal portion.

The device comprises first fixing members, operatively interposed between said at least one arm and said proximal portion,
wherein the first fixing members are configured to be activated when said at least one arm reaches the operative position to prevent forward and backward movement along said main direction of movement of the arm relative to the proximal portion, and wherein the first fixing members are configured to be deactivated to enable the movement of the arm from the operative position to the rest position.

## Description

The present invention relates to a device for inspecting a cavity of a patient, in particular an internal cavity of a patient, to be understood as a natural cavity, for example an anal and/or vaginal cavity, or to be understood as an artificial cavity made by the operator.

Hereinafter, precise reference will be made to the field of coloproctology, i.e., devices such as anoscopes, proctoscopes or rectoscopes, although the present invention may have other diagnostic and/or surgical applications.

The closest known art to the present invention is the inspection device of the same Applicant described in patent application EP3329834.

In accordance with such a publication, a dilator body is described which can be coupled in the rear with an adaptor for a camera and special lateral windows which enable manoeuvring surgical instruments inside the dilator body to act on the patient's mucosa, while the camera takes images of the operation for convenient viewing on a monitor. This allows the operator an excellent view and a high degree of surgical precision.

The Applicant has noted that the device described in the above patent application, although excellently functional, can be further improved in some respects.

In particular, for example when using lasers, the high precision required necessitates a resting point for the laser, which would otherwise have to be fully supported by the operator, making it difficult to achieve the necessary precision. However, having a rest seat for the fixed laser head may be inconvenient or interfere with the operator's view.

Although supports applied inside the dilator body to support surgical instruments are present in the prior art, they are unsuitable for the present application as their use would reduce the field of view and thus complicate the surgical procedure or alternatively require sacrificing the degree of precision of manoeuvrability of the laser head.

Therefore, the object of the present invention is to provide a device for inspecting a cavity of a patient which has a high degree of flexibility of use, in particular which enables use with a wide range of surgical instruments, including a laser tool, without obstructing an operator's view.

It is further an object of the invention to provide a device for inspecting a cavity of a patient which can be placed in an operative position in a stable manner so as to allow a high degree of surgical precision, in particular with the use of a laser tool, and which can be placed in a rest position when the use of the laser tool is not required so as to ensure that the operator has as much space to manoeuvre as possible.

The object is substantially achieved by a device for inspecting a cavity of a patient having the features expressed in one or more of the appended claims.

In particular, the inspection device according to the invention comprises a dilator body, extending along a longitudinal axis between a distal end and a proximal end, and a handle applied or fixed to the proximal end of the dilator body to allow an operator to manoeuvre the dilator body according to an insertion and/or rotation movement about the longitudinal axis.

The terms "distal" and "proximal" refer to, respectively, a region farther away from the operator (and thus closer to the patient) and closer to the operator in a condition of use of the device.

The device further comprises a distal portion having a substantially tubular shape defining a zone of dilation of an internal cavity of a patient and adapted to internally delimit an internal volume comprising a front opening to enable access to the patient's mucosa or tissue from such an internal volume.

The device further comprises a proximal portion, extending from the distal portion in a diverging fashion, comprising a rear access opening made on said proximal portion communicating with said internal volume.

The device further comprises at least one arm mounted on the proximal portion and comprising at least one rest seat configured to receive, resting upon it, a front surface of a laser head and to enable the movement thereof in such a way as to emit a linear laser beam through the front opening.

At least one arm is rotatable according to a main direction of movement relative to the proximal portion and is movable between an operative position, in which the arm externally embraces the proximal portion, and a rest position, in which the arm does not embrace such a proximal portion. The device further comprises first fixing members operatively interposed between at least one such arm and the proximal portion.

Such first fixing members are configured to be activated when at least one arm reaches the operative position to prevent forward and backward movements of at least one arm relative to the proximal portion along the main direction of movement.

Such first fixing members are configured to be deactivated to enable the movement of at least one arm from the operative position to the rest position.

Further features and advantages of the present invention will become more apparent from the indicative and thus non-limiting description of an embodiment of a device for inspecting a cavity of a patient made according to the present claims.

Such a description will be set out below with reference to the accompanying drawings, which are provided solely for illustrative and therefore non-limiting purposes, in which:
- Figure 1 shows a lateral view of a device for inspecting a cavity of a patient in accordance with the present invention in which an arm is arranged in an operative position;
- Figure 2 shows a lateral view of the device illustrated in Figure 1 for inspecting a cavity of a patient in accordance with the present invention in which one arm is arranged in a rest position;

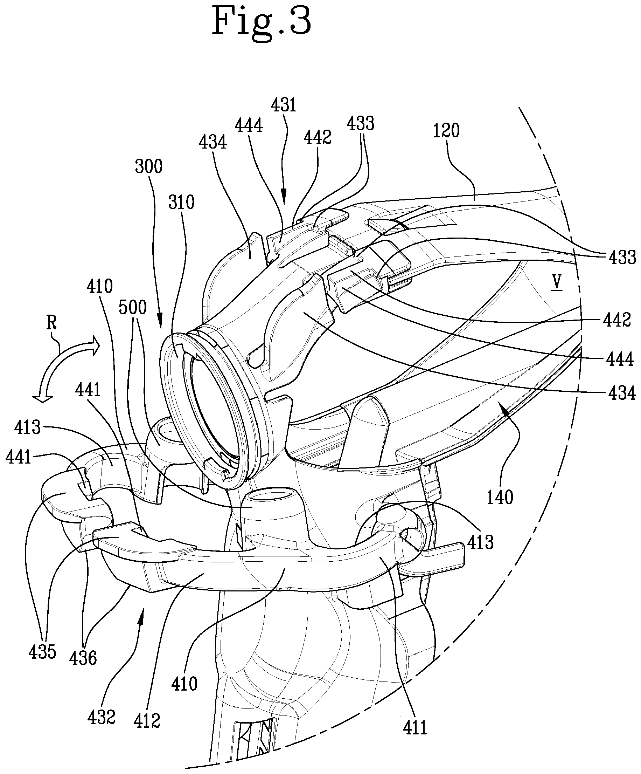

- Figure 3 shows an upper view of the device illustrated in Figure 1 for inspecting a cavity of a patient in accordance with the present invention in which at least one arm is arranged in a rest position;

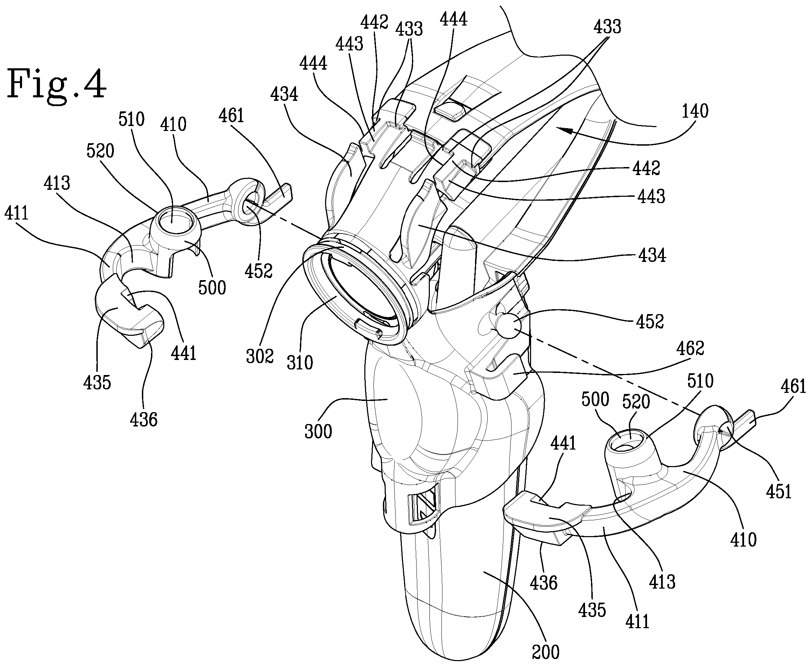

- Figure 4 shows a lateral view and rear view of the device illustrated in Figure 1 and the arm dismounted;

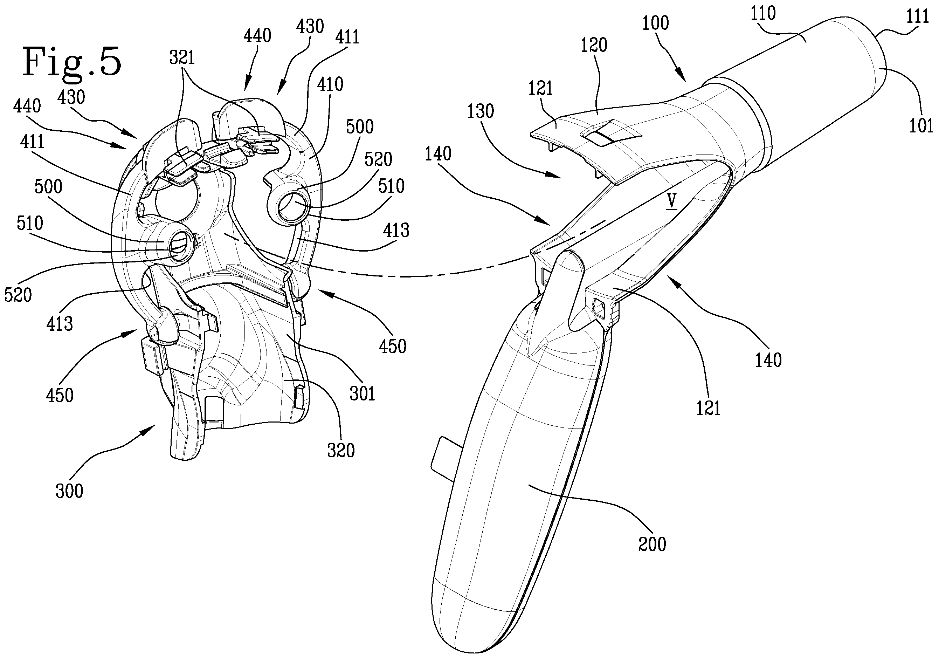

- Figure 5 illustrates a device according to the present invention with an adaptor body dismounted relative to the dilator body.

In accordance with the accompanying figures, an inspection device according to the invention is overall referred to with 1.

In the illustrated embodiment, the device 1 comprises a dilator body 100 and a handle 200 applied or fixed to the dilator body 100.

The dilator body 100 extends along a longitudinal axis "X" between a distal end 101 and a proximal end 102.

Preferably, the dilator body 100 and the handle 200 are made of plastic, more preferably transparent or semi-transparent.

In use, the handle 200 can assume different orientations, which in the present description will be defined as "downwards" so that the definition of "low" and "high" are to be understood as "in the extension region of the handle 200" and "in the direction opposite the handle 200". The definition of "lateral" is similarly applied, referring to the handle 200 normally facing downwards.

The handle 200 is applied or fixed to a proximal portion 120. Such a connection between the proximal portion 120 and the handle 200 can be made either integrally or with reversible or irreversible stable application. The handle 200 allows the operator to manoeuvre the dilator body 100 according to an insertion and/or rotation movement about the longitudinal axis "X".

The device 1 comprises a distal portion 110 having a substantially tubular shape defining a zone of dilation of an internal cavity of a patient and adapted to internally delimit an internal volume "V", comprising a front opening 111 to enable access to the patient's mucosa or tissue from the volume "V". Such mucosa or tissue can thus protrude inside the volume "V" or only remain exposed, depending on the case.

The definition "tubular" does not exclude the possibility that the outer shape of the distal portion 110 has a slightly tapered shape or provided with a section which is not perfectly circular (it could also be elliptical, oval or still others), since the specific shape of the distal portion 110 can be selected according to needs without departing from the same inventive concept of the invention.

The distal portion 110 thus extends along the longitudinal axis "X" and is shaped to delimit an internal volume "V" in the form of a longitudinal canal. The device 1 comprises the proximal portion 120, extending from the distal portion 110 in a diverging fashion, comprising a rear access opening 130 made on the proximal portion 120. Such a rear opening 130 is communicating with the internal volume "V".

In particular, the proximal portion 120 opens into such a rear access opening 130 facing the operator and arranged on the longitudinal axis "X". Such a rear access opening 130 thus allows visual and instrument access to the internal volume "V" and, therefore, to the mucosa accessible through the front opening 111 of the distal portion 110.

The device further 1 comprises at least one arm 410 mounted on the proximal portion 120 and comprising at least one rest seat 500 configured to receive, resting upon it, a front surface of a laser head and to enable the movement thereof in such a way as to emit a linear laser beam through the front opening 111.

A "rest seat" is understood as a generic abutment portion for a front portion of the laser head (or other instrument suitable for being held in abutment) to define at least longitudinal positioning abutment of the laser head. For this purpose, the rest seat 500 is arranged at least partially transverse to the longitudinal axis "X".

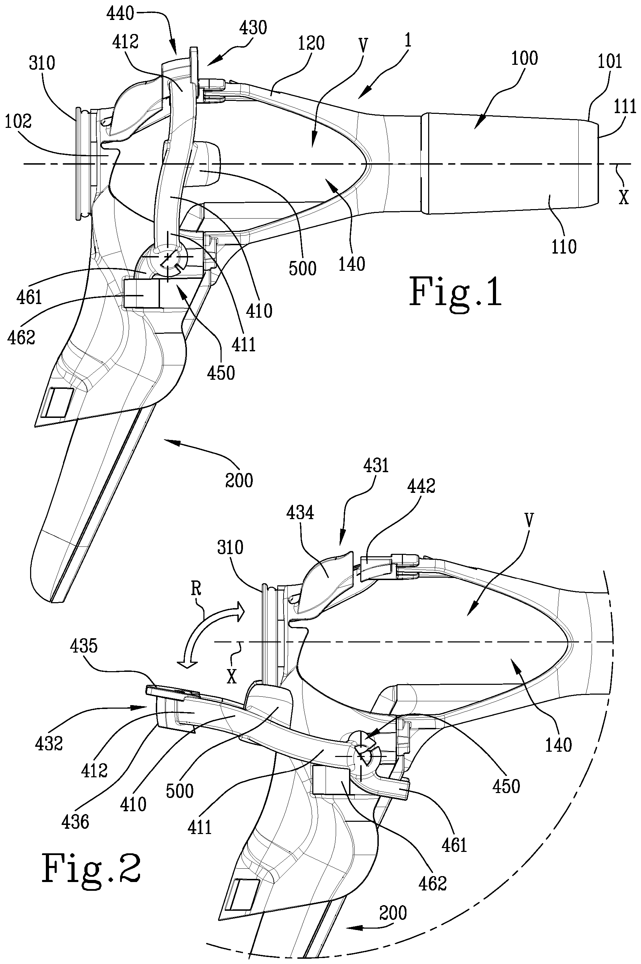

At least one arm 410 is rotatable according to a main direction of movement "R" relative to the proximal portion 120 and is movable between an operative position, as illustrated for example in Fig. 1, in which said at least one arm 410 externally embraces said proximal portion 120, and a rest position, as illustrated for example in Fig. 2, in which at least one arm 410 does not embrace said proximal portion 120.

The device 1 further comprises first fixing members 430 operatively interposed between the above-mentioned arm 410 and the proximal portion 120.

In particular, first fixing members 430 are configured to be activated when at least one arm 410 reaches the operative position to prevent forward and backward movement along the main direction of movement "R" of the arm 410 relative to the proximal portion 120 and to be deactivated to enable the movement of the arm from the operative position to the rest position. Movement "R" is intended as a rotation of the arm 410 aligned along a plane.

"Forward and backward movements" is intended as both main directions of movement "R" of the arm 410, as opposed to all the other movements such as, for example, transverse or lateral movements relative to the plane in which the main movement "R" occurs.

In particular, "forward" means the direction of movement when the arm 410 is approaching the operative position, while "backward" means the direction of movement when the arm 410 is moving away from the operative position.

In particular, the arm 410 comprises a first end 411 near which it is rotationally connected to the proximal portion 120 and a second end 412 near which first fixing members 430 are arranged.

The operative position envisages that the second end 412 is connected to the proximal portion 120 by means of such first fixing members 430. Instead, the rest position envisages that the second end 412 to be disconnected from the proximal portion 120 and moved away relative to the proximal position 120.

The first fixing members 430 comprise a first component 431, located on the proximal end 102, and a second component 432, located on the arm 410.

The first component 431 comprises a first stop, configured to prevent forward movements of the second component 432 along the main direction of movement "R", and a second stop, configured to prevent backward movements of the second component 432 along the main direction of movement "R".

The first fixing members 430 define a reversible snap-locking system activatable towards at least one arm 410 in its rotation towards the operative position and manually deactivatable to enable the passage of the arm 410 towards the rest position.

In particular, the first stop is a front stop 433, opposed to forward movement, relative to the main direction of movement "R" and the second stop is a rear stop 434, opposed to backward movement, relative to the main direction of movement "R". The front stop 433 is rigid. The reversible snap-locking system, on the other hand, comprises the second component 432 and the rear stop 434, the latter of which is configured to deform elastically relative to the second component 432 and the first front 433. Such a deformation occurs during the passage of the arm 410 towards the operative position, to then return to the initial, undeformed position, preventing forward and backward movements along the main movement direction "R" of the arm 410 relative to the proximal portion 120.

In particular, the rear stop 434 is shaped like a step which is at least partially flexible relative to the proximal portion 120.

Even more in particular, the rear stop 434 has a greater extent in height relative to the proximal portion than the front stop 433.

The device 1 further comprising second fixing members 440, operatively interposed between the arm 410 and the proximal portion 120.

Such second fixing members 440 are configured to prevent the lateral movements of the arm 410 relative to the proximal portion along a direction perpendicular to the main direction of movement "R".

In particular, such second fixing members 440 comprise a channel 441 suitable for receiving a guide 442 in the rotation of said at least one arm 410 towards said operative position, wherein such a guide 442 comprises a first and a second lateral wall 443 and 444, parallel and opposite each other, said first and second lateral walls 443 and 444 being configured to abut laterally against the inner surface of the channel 441, thereby preventing the lateral movements of the arm 410 relative to the proximal portion 120 along a direction perpendicular to the main direction of movement "R".

Preferably, the channel 441 is made longitudinally substantially in an hourglass shape, comprising widened ends suitable for facilitating the entry and exit of the guide 442 relative to the channel 441.

In particular, in the embodiment illustrated in the present figures, the channel 441 is transversely "U"-shaped and oriented along the main direction of movement "R", while the guide 442 is shaped cantilevered relative to the proximal portion 120, longitudinally linear and oriented along the main direction of movement "R" and compatible in shape and internally sliding along the channel 441.

Preferably, the aforementioned front stop 433 and rear stop 434 are arranged respectively in the front and rear relative to the guide 442, all three being arranged in succession along the main direction of movement "R".

The second component 432 in turn comprises a front surface 435 and a rear surface 436, arranged transversely relative to the main direction of movement "R". Such front surface 435 and rear surface 436 being configured to abut with the front stop 433 and the rear stop 434, respectively, when the arm 410 is in the operative position.

Advantageously, in such an embodiment, the first fixing members 430 and the second fixing members 440 simultaneously and synergistically allow a stable coupling, while the first component 431 effectively allows the arm 410 to slide and its relative fixing in the operative position, ensuring a high stability both during and after the arrangement of the arm 410 in the operative position.

The first fixing members 430 and the second fixing members 440 are preferably arranged laterally on the proximal portion 120.

Even more preferably, the first fixing members 430 and the second fixing members 440 are preferably arranged high on the proximal portion 120.

In the present embodiment, at least one arm 410 is connected to the proximal portion 120 by means of an at least partially spherical hinge 450. In particular, the hinge 450 comprises a recess 451 which is at least semi-spherical and arranged on the arm 410, and an engagement head 452 which is at least semi-spherical and arranged to extend the proximal portion 120. The engagement head 452 is reversibly connectable to the recess 451 and is configured to allow an at least partially spherical movement relative to such a recess 451.

Preferably the hinge 450 is arranged low relative to the proximal portion 120, preferably laterally near the junction point between the dilator body 100 and the handle 200.

Advantageously, the arm by means of the recess 451 allows a reversible connection and disconnection to the proximal portion 120 of the dilator body 100, being able to be added or removed as required. Furthermore, thanks to the movement with at least partially spherical mobility, the arm 410 can be moved easily between the operative and rest position. Despite such movement with at least partially spherical mobility, the arm 410 substantially follows a main direction of movement "R" to arrange itself in the operative position, rotating by a certain section substantially along a plane. Such a section is given by the length of the guide 442, when the guide is engaged inside the channel 441.

In particular, according to a first embodiment, the main movement direction "R" lies on a plane parallel to the longitudinal axis "X". That is, the arm 410 rotates longitudinally relative to the longitudinal axis "X". Alternatively, according to a second embodiment, the main direction of movement "R" lies in a plane which is perpendicular relative to the longitudinal axis "X". That is, the arm 410 rotates perpendicularly relative to the longitudinal axis "X".

Alternative embodiments can envisage a main direction of movement "R" along an inclined plane which is intermediate relative to the first embodiment and the second embodiment.

Substantially, by coupling and decoupling first and second components 431, 432 of the first fixing members 430, the arm 410 reversibly switches between the operative and rest position. Preferably, once in the rest position, however, the arm 410 can be moved and arranged in various intermediate rest positions, chosen according to the convenience of the operator to view the exposed tissues and perform surgical operations by means of the rear opening 130 and through the volume "V" and the front opening 111.

In order for the arm 410 to be further stabilised in the operative position, the arm 410 comprises near the first end 411 an extension 461 and the proximal portion 120 comprises a housing 462 configured to accommodate the extension 461 when the arm 410 is in the operative position.

In particular, the housing 462 is shaped like a protruding "C" relative to the proximal portion 120 configured to accommodate the extension 461 and participating in preventing any displacements of the arm 410 when in the operative position.

The extension 461 is substantially shaped like an elongation of the arm 410.

Preferably, the housing 462 is arranged substantially near the junction point between the dilator body 100 and the handle 200.

The arm 410 preferably has at least one curved portion 411 defining a concavity 413 and the rest seat 500 is arranged on such a respective curved portion 411.

Preferably, the arm 410 externally embraces the proximal portion 120 of the dilator body 100 along such a concavity 413.

According to the embodiment illustrated in the present figures, the rest seat 500 is arranged inside said concavity 413 and outside said proximal portion 120.

Alternative embodiments may envisage that the rest seat 500 is arranged outside such a concavity 413 in a manner opposite the concavity 413 relative to said arm 410.

Preferably, the proximal portion 120 has at least one lateral window 140 at which the rest seat 500 is arranged such that the rest seat 500, the front opening 111 and the lateral window 140 are joined by a linear segment when the arm 410 is in the operative position.

Preferably, the rest seat 500 is arranged overlapping the respective lateral window 140 of the adaptor body 300 in a radially external position relative to such a side window 140.

However, non-illustrated embodiment variants are possible wherein the rest seat 500 is arranged on the respective arm 410 in such a way that it is in a position radially inside the respective lateral window 140 or straddles the lateral windows 140 itself.

The rear opening, the volume "V" and the front opening are preferably aligned on the axis "X". Advantageously, it follows that the position of the seat 500 is such that it does not obstruct an operator's view when performing operations on the tissues.

The window 140 preferably extends along the entire proximal portion, thus from the rear opening 130 of the proximal portion 120 up to the distal portion 110.

Alternatively, the lateral window 140 could be less extended or more extended, in the latter case also affecting part of the distal portion 110.

The lateral window 140 is preferably defined by a rear recess which interrupts the profile of the rear edge of the proximal portion 120 and then extends from the rear opening of the proximal portion 120 towards the distal portion 110.

The lateral window 140 defines further zones of visual and instrument access to the internal volume "V".

According to the present embodiment, with reference to Fig. 1, in the operative position, at least one arm 410 is near the front opening 111 and/or a lateral opening 140 and the rest seat 500 is usable by the operator to insert and manoeuvre a laser head.

In the rest position, with reference to Fig. 2, at least one arm 410 is overturned, preferably near or resting against the handle 200, placing the rest seat 500 in a position away from the dilator body 100, in particular from the front opening 111 and from the side opening 140. In this latter position, the operator can exploit the free front opening 111 so that further surgical instruments can be inserted by means of the lateral window 140. According to the embodiment illustrated in the present figures, the device 1 comprises an adaptor body 300 stably applicable to the dilator body 100 near the rear opening 130.

The arm 410 is connected to such an adaptor body 300.

The adaptor body 300 is stably applicable to the dilator body 100, in particular to the handle 200 by means of a first connecting portion 320, preferably by means of snap connection around the handle 200, and to the proximal portion 120 by means of a second connecting portion 321, preferably comprising means for interlocking connection to the proximal portion 120.

That is, the adaptor body 300 is configured for the axially sliding reversible application (parallel to the longitudinal axis "X" on the proximal end 120 of the dilator body 100 preferably with a snap lock (with stop abutment) at one or more possible pre-established positions.

The adaptor body 300 comprises a seat for a camera 310 arranged aligned with the axis "X" or, in other words, capable of displaying the exposed tissue from the front opening 111 by means of the rear opening 130.

In particular, the adaptor body 300 has a first, front, end 301 which can be reversibly connected to the dilator body 100, in particular to the proximal portion 120, and a second, rear, end 302 opposite the first and preferably provided with a connecting means (known, e.g., snap-on or screw-on) to an operative accessory, in particular to a camera.

Preferably, the adaptor body 300 has a tapered shape in the direction of the second end 302, where such a said tapered shape may only affect the end portion of the adaptor body 300 facing the second end 302, in particular in the area of the first connecting portion 320, which is concave in shape and/or substantially counter-shaped to a corresponding portion of the handle 200.

According to the embodiment illustrated in the present figures, the device 1 preferably comprises a pair of arms 410, at least one of the two arms 410, preferably both, having a respective rest seat 500 and the arms 410 configured to embrace opposite symmetrical sides of the proximal portion 120.

Preferably, the proximal portion 120 has two opposite lateral windows 140, which preferably extend substantially along the entire axial extension of the proximal portion 120.

In the illustrated embodiment, therefore, the proximal portion 120 is substantially defined by two opposite lobes 121, one upper and one lower, which are connected to each other with a desired radius of curvature at the area of union between the proximal portion 120 and the distal portion 110.

The handle 200 is applied or fixed at one of the aforementioned lobes 121. Where envisaged, the adaptor body 300 is stably applicable in a reversible manner to the dilator body 100 at the two lobes 121.

Preferably, the adaptor body 300 also has a pair of opposite lateral windows 310, intended in use (i.e., after connection with the dilator body 100) to join with the aforementioned lateral windows 140 of the proximal portion 120.

The rest seat 500 is preferably defined by a stop wall 510 in the shape of a spherical dome, thus with a closed extension, having a central opening 520. The central opening 520 is intended for the passage of the laser beam and is preferably circular.

With reference to the laser head, it is configured to emit a laser beam and has a means for connection to an electrical or laser source. The front surface of the laser head has a spherical shape which can be engaged in a resting relationship with each rest seat 500.

In use, once the dilator body 100 has been inserted in position in the patient's cavity, the operator can intervene on the mucosa by means of surgical instruments through at least the lateral windows 140 and/or the rear opening 130 and/or proceed with an operation by means of a laser by arranging the laser head rotationally resting inside one of the rest seats 500 and rotating the laser head if necessary to move the laser beam, directing it to the desired region of the mucosa.

The present invention thus enables the proposed objects to be achieved, allowing a high flexibility of use with high accuracy thanks to the versatile support of the arm 410, capable of being flexibly but firmly arranged or available in an operative position in which an operator can precisely rest and direct the laser beam emitted by the laser head.

## Claims

1. A device (1) for inspecting a cavity of a patient, comprising:
- a dilator body (100) extending along a longitudinal axis (X) between a distal end (101) and a proximal end (102);
- a handle (200) applied or fixed to said dilator body (100);
wherein the dilator body (100) comprises:
- a distal portion (110) having a substantially tubular shape defining a zone of dilation of an internal cavity of a patient and adapted to internally delimit an internal volume (V) comprising a front opening (111) to enable access to the patient's mucosa or tissue from said volume (V);
- a proximal portion (120), extending from the distal portion (110) in a diverging fashion, comprising a rear access opening (130) made on said proximal portion (120) communicating with said internal volume (V);
- at least one arm (410) mounted on the proximal portion (120) and comprising at least one rest seat (500) configured to receive, resting upon it, a front surface of a laser head and to enable the movement thereof in such a way as to emit a linear laser beam through said front opening (111);
wherein said at least one arm (410) is rotatable according to a main direction of movement (R) relative to said proximal portion (120) and is movable between an operative position, in which said at least one arm (410) externally embraces said proximal portion (120), and a rest position, in which said at least one arm (410) does not embrace said proximal portion (120);
- first fixing members (430), operatively interposed between said at least one arm (410) and said proximal portion (120),
wherein said first fixing members (430) are configured to be activated when said at least one arm (410) reaches the operative position to prevent forward and backward movements of said at least one arm (410) relative to the proximal portion along said main direction of movement (R), and wherein said first fixing members (430) are configured to be deactivated to enable the movement of said at least one arm (410) from the operative position to the rest position.

2. The device (1) for inspecting a cavity of a patient according to claim 1, wherein said first fixing members (430) comprise a first component (431), located on said proximal end (102), and a second component (432), located on said arm (410);
wherein said first component (431) comprises a first stop, configured to prevent forward movements of said second component (432) along said main direction of movement (R), and a second stop, configured to prevent backward movements of said second component (432) along said main direction of movement (R).

3. The device (1) for inspecting a cavity of a patient according to claim 2, wherein said first fixing members (430) define a reversible snap-locking system activatable towards said at least one arm (410) in its rotation towards said operative position and manually deactivatable to enable the passage of said at least one arm (410) towards said rest position.

4. The device (1) for inspecting a cavity of a patient according to claim 3, wherein said first stop is a front stop (433) relative to said main direction of movement (R) and said second stop is a rear stop (434) relative to said main direction of movement (R), wherein said front stop (433) is rigid and wherein said reversible snap-locking system comprises said second component (432) and said rear stop (434), which is configured to deform elastically relative to the second component (432) and to the front stop (433) in the passage of said at least one arm (410) towards said operative position, in order to subsequently return into the initial position and prevent the forward and backward movements of said at least one arm (410) relative to the proximal portion along said main direction of movement (R).

5. The device (1) for inspecting a cavity of a patient according to claim 4, wherein said rear stop (434) is shaped like a step that is at least partly flexible relative to said proximal portion (120).

6. The device (1) for inspecting a cavity of a patient according to claim 5, wherein said rear stop (434) has a greater extent in height relative to the proximal portion than the front stop (433).

7. The device (1) for inspecting a cavity of a patient according to one or more of the preceding claims, comprising second fixing members (440), operatively interposed between said at least one arm (410) and said proximal portion (120), wherein said second fixing members (440) are configured to prevent the lateral movements of said at least one arm (410) relative to the proximal portion (120) along a direction perpendicular to said main direction of movement (R).

8. The device (1) for inspecting a cavity of a patient according to claim 7, wherein said second fixing members (440) comprise a channel (441) suitable for receiving a guide (442) in the rotation of said at least one arm (410) towards said operative position, wherein said guide (442) comprises a first and a second lateral wall (443 and 444), parallel and opposite each other, said first and second lateral walls (443 and 444) being configured to abut laterally against the inner surface of the channel (441), thereby preventing the lateral movements of said at least one arm (410) relative to the proximal portion (120) along a direction perpendicular to said main direction of movement (R).

9. The device (1) for inspecting a cavity of a patient according to claim 8, wherein said channel (441) is made longitudinally substantially in an hourglass shape, comprising widened ends suitable for facilitating the entry and exit of the guide (442) relative to the channel (441).

10. The device (1) for inspecting a cavity of a patient according to one or more of the preceding claims, wherein said at least one arm (410) is connected to the proximal portion (120) by means of an at least partially spherical hinge (450).

11. The device (1) for inspecting a cavity of a patient according to claim 10, wherein said main direction of movement (R) lies in a plane parallel to said longitudinal axis (X).

12. The device (1) for inspecting a cavity of a patient according to claim 10, wherein said main direction of movement (R) lies in a plane perpendicular to said longitudinal axis (X).

13. The device (1) for inspecting a cavity of a patient according to claim 11 or 12, wherein said arm (410) comprises an extension (461) and said proximal portion (120) comprises a housing (462) configured to accommodate said extension (461) when said at least one arm (410) is in the operative position.

14. The device (1) for inspecting a cavity of a patient according to any one of the preceding claims, wherein said arm (410) is connected to an adaptor body (300) stably applicable to the proximal portion (120) of the dilator body (100) by means of at least a first connecting portion (320) and comprising a seat for a camera (310) disposed aligned with said axis (X).

15. The device (1) for inspecting a cavity of a patient according to any one of the preceding claims, comprising a pair of arms (410), at least one of the two arms (410) having a respective rest seat (500) and the arms (410) configured to embrace opposite symmetrical sides of the proximal portion (120).
